# EUROPÄISCHE PATENTSCHRIFT

(11) **EP 0 785 860 B1**
(45) Veröffentlichungstag und Bekanntmachung des Hinweises auf die Patenterteilung: **27.01.1999**
(21) Anmeldenummer: 95935938.1
(22) Anmeldetag: 15.10.1995
(51) Int. Cl.: B30B 11/00, A61J 3/10, A61M 15/00

(54) **VERFESTIGTER ARZNEISTOFFVORRAT FÜR DIE ERZEUGUNG VON INHALIERBAREN ARZNEISTOFFPARTIKELN**
COMPACTED MEDICAMENT SUPPLY FOR GENERATING INHALABLE MEDICAMENT PARTICLES
RESERVE DE MATIERES MEDICINALES SOLIDIFIEES SERVANT A GENERER DES PARTICULES DE MATIERES MEDICINALES A INHALER

(30) Priorität: 15.10.1994 DE 4436854
(43) Veröffentlichungstag der Anmeldung: 30.07.1997
(73) Patentinhaber: GGU Gesellschaft für Gesundheits- und Umweltforschung mit beschränkter Haftung, 82169 Puchheim (DE)
(72) Erfinder: HEIDE, Helmut, D-65779 Kelkheim - 2 (DE); PABST, Joachim, D-64354 Reinheim (DE); BURGSCHAT, Hans, D-55124 Mainz (DE)
(74) Vertreter: Roesner, Werner
(86) Internationale Anmeldenummer: EP9504049
(87) Internationale Veröffentlichungsnummer: WO9611795

(56) Entgegenhaltungen:
- WO-A-94/00291
- FR-A- 1 562 890
- FR-A- 2 273 206
- US-A- 4 370 120

## Beschreibung

Die vorliegende Erfindung betrifft einen verfestigten Arzneistoffvorrat für die Erzeugung von inhalierbaren Arzneistoffpartikeln mittels einer Abtragemittel aufweisenden Dosiereinrichtung.

Derartige Arzneistoffvorräte sind aus WO 93/24165 bekannt und werden in Aerosolgeneratoren eingesetzt, die eine Abtragemittel aufweisende Dosiereinrichtung, beispielsweise in Form einer angetriebenen Stirnfräse, aufweisen, um Arzneistoffpartikel von dem Arzneistoffvorrat abzutragen und hierdurch ein Aerosol zu erzeugeen, das im Augenblick seiner Generierung zur oralen oder nasalen Inhalation freigesetzt wird.

Aus der DE 40 27 390 AI ist ein Inhalationsgerät bekannt, bei dem mittels einer Bürste von einer Tablette staubförmige Partikel abgetragen werden sollen, wobei die geometrischen Formen dieser Tablette variieren können.

Die EP 0 407 028 A2 offenbart ebenfalls einen Aerosolgenerator, der einen verpreßten Körper eines Inhalationsmedikamentes enthält. Als Preßdrücke für das Verpressen des Arzneistoffvorrates ist ein sogenannter leichtverdichteter Druckbereich zwischen 1 x 10⁴ bis 15 x 10⁴ N/m² sowie ein starkverdichteter Druckbereich zwischen 30 x 10⁴ und 150 x 10⁴ N/m² angegeben. Jedoch ist selbst dieser höhere Druckbereich derart niedrig angesetzt, daß ein handhabbares Gefüge nur dadurch existieren kann, daß der Arzneistoffvorrat in einen zylindrischen Behälter gedrückt wird und von diesem zusammengehalten wird. Aufgrund der relativ losen Kompaktierung des Arzneistoffvorrats ist jedoch aufgrund innerer Reibungseffekte in der Schüttung und der Reibung zwischen Arzneistoffvorrat und Behälterwandung eine homogene Druckverteilung in der Arzneistoffschüttung nicht zu erreichen. Folglich weisen derartige Arzneistoffvorräte Dichtegradienten von mehr als 40% auf, die sich negativ auf die Dosiergenauigkeit auswirken (vgl. hierzu: B. Charlton and J.M. Newton, Application of Gamma-Ray Attenuation to the Determination of Density Distribution within Compacted Powders, Powder Technology, 41 (1985), 123 bis 134).

Da nach einer Veröffentlichung des Anmelders der EP 0 407 028 A2 die Dichten des Arzneistoffvorrates bei ca. 0,8 bis 0,9 g/cm⁻³ liegen, läßt sich für den verdichteten Arzneistoffvorrat überschlägig eine Raumdichte von nur etwa 50% der theoretischen Dichte berechnen. Dies bedeutet, daß ein derartig hochporöser Arzneistoffvorrat nicht nur sehr locker und inhomogen ist, sondern infolge der großen offenen Porosität zwangsläufig der Agglomeration und der Alterung durch die atmosphärischen Einflußfaktoren, wie Luftfeuchtigkeit und dergleichen, unterliegt.

Aus der PCT/EP 93/01158 ist ein verfestigter Arzneistoffvorrat bekannt, wobei in dieser Veröffentlichung auf die Bedeutung der strukturellen und chemischen Homogenität des verfestigten Arzneistoffvorrats für die Dosiergenauigkeit hingewiesen wird. Zur Erzielung eines derartigen Gefügezustandes wird dort das isostatische Pressen vorgeschlagen, welches ein aus der Pulvermetallurgie bekanntes Preßverfahren in flexiblen Formen ist. Hierbei wird eine Pulverschüttung, die sich z.B. in einer Gummiform befindet, von außen mit einer Druckflüssigkeit allseitig und somit isostatisch verdichtet. Auch werden in dieser Veröffentlichung neben dem isostatischen Pressen andere Formgebungsverfahren vorgeschlagen, wie z.B. das Spritzgießen plastifizierter Massen.

Eine Vorrichtung zum isostatischen Pressen zur Herstellung von Dichtungen und dgl. geht aus US - A - 4 370 120 hervor. Hierbei findet das Pressen nur radial statt und wurde daher nicht zur Bildung von Preßtexturen im Bereich der Endfläche eines Arzneistoffvorrates führen.

Gemäß WO - A - 94 00 291 ist ein Preßverfahren bekannt,bei dem mittels Druckluft eine expandierbare Druckmembrane auf eine Pulverschüttung aufgepresst wird, so daß in einer außenliegenden Form aus der Pulverschüttung ein Ring gepresst wird. Die Druckübertragung auf die Pulverschüttung erfolgt also von innen nach außen.

Im Rahmen weiterer Untersuchungen an verfestigten Arzneistoffvorräten hat sich gezeigt, daß die Anforderungen hinsichtlich der Gefügequalität die bisher bekannten Vorstellungen weitaus überschreiten. Es hat sich herausgestellt, daß sich bei der Abtragung mittels einer Abtragemittel aufweisenden Dosiereinrichtung, wie sie beispielweise aus der WO 93/24165 bekannt ist, etwaige Dichteschwankungen signifikant auf die generierte Aerosolmenge auswirken.

Auch wurde beobachtet, daß isostatisch gepreßte Arzneistoffvorräte an der Abtragungsstirnfläche zunächst unerklärliche Dosierungsschwankungen aufwiesen, die durch integral meßbare Dichte- und stoffliche Inhomogenitäten nicht zu erklären waren. Eine genauere Untersuchung dieser Phänomene ergab nun, daß sich in diesen Randbereichen Gefügefehler, wie Preßtexturen und Mikrorisse befinden, die bei einer Abtragung zu Abplatzungen größerer Gefügebereiche und somit zu Dosierungsschwankungen führen können.

Es hat sich nunmehr herausgestellt, daß solche Gefügefehler auf das isostatische Pressen zurückzuführen sind. Eine ideale isostatische Druckübertragung auf eine Pulverschüttung findet strenggenommen nur bei kugelförmigen oder zumindest der Kugelgeometrie nahekommenden Formen statt. Weicht jedoch die Formgeometrie von der isometrischen Form ab, was z.B. bei einem ringförmigen Abtragungskörper für Inhalationszwecke der Fall ist, so erfolgt die Druckübertragung nicht mehr rein isostatisch sondern gerichtet. Wird beispielsweise eine solche Inhalator-Ringtablette isostatisch gepreßt, so überlagern sich im Bereich der Endflächen radiale und axiale Kräfte, die in diesem kritischen Bereich der Stirnflächen zu Preßtexturen führen, insbesondere da eine so gestaltete Form gegenüber dem hydrostatischen Druck unterschiedliche Steifigkeit und somit eine unterschiedliche Verformbarkeit in der Druckaufbauphase besitzt. Die Folge ist eine primäre Verdichtungsphase in radialer Richtung und eine darauffolgende Nachverdichtung des schon vorverfestigten Pulvergefüges in axialer Richtung, was zu den erwähnten Texturen und Mikrorissen im Gefüge der Ringtablette führt. Bei den bekannten Anwendungen des isostatischen Pressens im Bereich der Pulvermetallurgie und der Keramik spielen derartige Erscheinungen im allgemeinen keine größere Rolle, da die auftretenden Gefügefehler durch die üblicherweise durchgeführten nachfolgenden Sintervorgänge ausheilen und keine Mängel hinterlassen. Diese Möglichkeit ist jedoch bei einem Arzneistoffvorrat, der ohne eine solche Nachbehandlung verwendet wird, nicht gegeben.

Ferner hat sich herausgestellt, daß auch beim isostatischen Pressen eines Arzneistoffvorrates die eingangs erwähnten Reibungseffekte innerhalb einer Pulverschüttung auftreten, wenngleich nicht in dem Maße wie bei unidirektionalen Preßtechniken. Der Druckverlust im Inneren der Pulverschüttung ist auch beim isostatischen Pressen umso größer, je dickwandiger das Formteil in Richtung der Druckeinwirkung ist. Dies bedeutet, daß auch beim isostatischen Pressen einer Ringtablette Innerhalb der Zylinderwandung in radialer Richtung und axialer Richtung Dichtegradienten auftreten, die im Bereich der Stirnfläche komplett geformte Zonen gleicher Dichte bzw. Texturen bewirken.

Es ist die der Erfindung zugrundeliegende Aufgabe, einen verfestigten Arzneistoffvorrat zu schaffen, der bei einer Abtragung über den gesamten Verbrauchsbereich reproduzierbar und gleichbleibend die gewünschten Partikel abgibt.

Die Lösung dieser Aufgabe erfolgt erfindungsgemäß durch die Merkmale des Patentanspruchs 1 und insbesondere dadurch, daß das Gefüge des Arzneistoffvorrates durch Aufbringen eines radialen Preßdruckes von außen auf einen im Zentrum des Arzneistoffvorrates positionierten Kern hergestellt ist.

Der erfindungsgemäß vorgesehene Arzneistoffvorrat läßt sich in einem Aerosolgenerator anordnen, wie er aus der WO 93/24165 der vorliegenden Anmelder bekannt ist. Auf die in dieser Veröffentlichung beschriebene Vorrichtung wird hiermit ausdrücklich Bezug genommen und diese wird durch Bezugnahme zum Gegenstand der vorliegenden Anmeldung gemacht. Bei Abtragung der Aerosoldosis durch die in axialer Richtung einwirkende Stirnfräse ist durch den erfindungsgemäßen Arzneistoffvorrat eine insgesamt sehr geringe, insbesondere aber koaxiale Dichteverteilung vorgegeben, so daß kein Einfluß auf die erzeugte Aerosolmenge und -qualität gegeben ist.

Vorteilhafte Ausführungsformen des erfindungsgemäßen Arzneistoffvorrates sind durch die Unteransprüche 2 bis 8 gekennzeichnet. Durch das erfindungsgemäße Aufbringen des Preßdruckes - verglichen zum isostatischen Pressen - führt die Erfindung überraschenderweise zu hervorragenden Eigenschaften des verfestigten Arzneistoffvorrates. Da der Preßdruck in radialer Richtung aufgebracht wird, besitzt der so erzeugte Arzneistoffvorrat infolge der oben beschriebenen inneren Reibung einen Dichtegradienten, der streng radial ausgerichtet ist, d.h. die Zonen gleicher Dichte verlaufen parallel und konzentrisch zur Längsachse des Arzneistoffvorrates.

Nach einer weiteren Ausbildung der Erfindung kann der Arzneistoffvorrat einen kreisringförmigen Querschnitt aufweisen.

In der Praxis besonders vorteilhaft ist die Anwendung der Erfindung auf Ringtabletten. Hierbei wird ebenfalls ein Preßdruck in im wesentlichen radialer Richtung gegen einen feststehenden Kern gerichtet.

Durch diese geometrischen Formen ergibt sich durch Anwendung radialen Preßdruckes ein besonders vorteilhaftes Gefüge mit minimalem Dichtegradienten in radialer und axialer Richtung. Hierbei kann der Dichtegradient nach einer weiteren vorteilhaften Ausführungsform der Erfindung senkrecht zur Richtung der Abtragung nicht mehr als 0,3 % und in Richtung der Abtragung nicht mehr als 0,05 % betragen. Derartig niedrige Dichtegradienten konnten bislang bei Arzneistoffvorräten für die Erzeugung von inhalierbaren Arzneistoffpartikel mittels einer Dosiereinrichtung nicht verwirklicht werden. Die Preßdrücke für die erfindungsgemäßen Arzneistoffvorräte liegen zwischen 50 und 500 MPa.

Nach einem weiteren Aspekt der Erfindung betrifft diese ein Verfahren zur Herstellung eines verfestigten Arzneistoffvorrates für die Erzeugung von inhalierbaren Arzneistoffpartikeln mittels einer Abtragemittel aufweisenden Dosiereinrichtung, wobei auf den Arzneistoff ein Preßdruck aufgebracht wird. Erfindungsgemäß wird hierbei der Preßdruck radial von außen auf einen im Zentrum des Arzneistoffvorrates positionierten Kern gerichtet. Durch ein solches Herstellungsverfahren werden die eingangs erläuterten vorteilhaften Eigenschaften eines Arzneistoffvorrates erzielt, der für die Erzeugung von inhalierbaren Arzneistoffpartikeln verwendet wird.

Entsprechend der gewünschten Form des verfestigten Arzneistoffvorrats wird dieser vorgeformt dem Preßdruck ausgesetzt. Im Falle einer Ringtablette wird der Arzneistoff in Pulverform oder vorverdichtet um einen festen Kern angeordnet und dann durch radialen Druck gegen den Kern gepreßt. Nach dem Pressen wird vorzugsweise der Kern entfernt und die Ringtablette entnommen.

Der Arzneistoffvorrat kann zunächst als stabartiger Körper, beispielsweise in einer Länge von 200 mm geformt und anschließend senkrecht zur axialen Erstreckung getrennt werden. Dieses Verfahren ermöglicht eine äußerst effiziente Herstellung einer Vielzahl von Arzneistoffvorräten mit den gewünschten Eigenschaften, die in einen zugeordneten Aerosolgenerator eingesetzt werden können.

Nachfolgend wird die Erfindung unter Bezugnahme auf die beigefügte Zeichnung rein beispielhaft anhand einer vorteilhaften Ausführungsform beschrieben. Es zeigen:

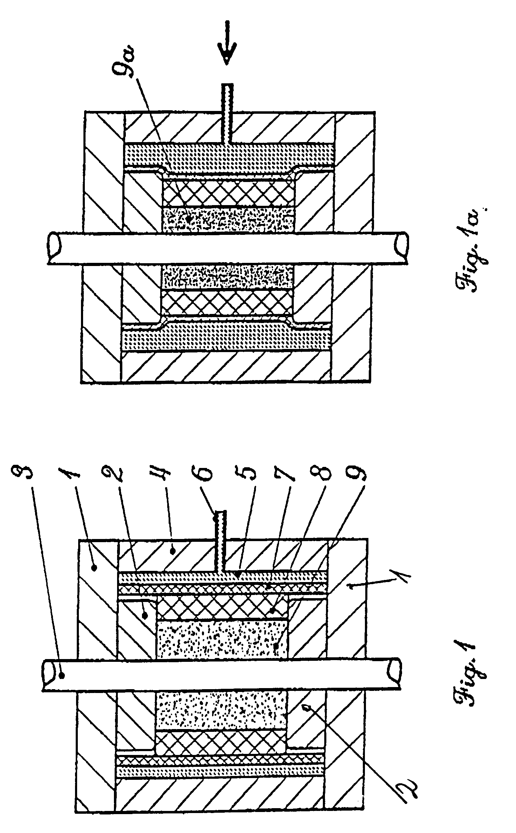

- Fig. 1: eine schematische Querschnittsansicht einer Vorrichtung zur Herstellung eines verfestigten Arzneistoffvorrates im drucklosen Zustand; und
- Fig. 1a: die Vorrichtung von Fig. 1 bei Beaufschlagung mit Preßdruck.

Die in Fig. 1 dargestellte Presse zur Herstellung eines verfestigten Arzneistoffvorrates durch Aufbringen eines Preßdruckes auf den Arzneistoff 9 weist einen Preßraum auf, der durch zwei feststehende und parallel beabstandete Platten 2 und eine flexible Preßmatritze 8 gebildet ist. Hierbei sind die Flächen der Platten 2 und der Preßmatritze 8, die in den Preßraum weisen, senkrecht zueinander angeordnet. Das äußere Gehäuse der in Fig. 1 dargestellten Preßkammer ist durch eine obere Gehäuseplatte und eine untere Gehäuseplatte 1 gebildet, die durch die Schließkraft einer nicht dargestellten hydraulischen Presse fest auf den Ringmantel 4 gedrückt wird. Die Schließkraft der hydraulischen Presse ist dabei sehr viel höher als der Öldruck im Inneren der Preßkammer auf die Preßmatritze, so daß ein Austreten der hydraulischen Druckflüssigkeit unmöglich ist. Durch den Mittelpunkt der beiden Gehäuseplatten erstreckt sich ein Stahlkern 3, der auch durch die Platten 2 und den Preßraum geführt ist.

Wie Fig. 1 zeigt, umgibt die flexible Preßmatritze 8 radial den Ringraum, der an der Ober- und der Unterseite durch die Platten 2 begrenzt wird. Hierbei ist die flexible Preßmatritze 8 so dimensioniert, daß sich diese zwischen den beiden parallelen Platten 2 erstreckt. Die Preßmatritze 8 ist ferner von einer flexiblen Druckmembran 7 radial umgeben, die sich in axialer Richtung zwischen der oberen und der unteren Gehäuseplatte 1 erstreckt und die eine Abdichtung gegenüber dem Druckmedium 5, beispielsweise Hydrauliköl, bewirkt, das über einen Anschluß 6 von außen zugeführt wird.

Zur Herstellung des verfestigten Arzneistoffvorrates wird zunächst der Arzneistoff in den Preßraum 8 eingebracht, wobei dieser eine granulierte Trägerstoff/Wirkstoffmischung sein kann, die beispielsweise durch Sprühgranulation oder trockenes Zwangsmischen sehr gut rieselfähig gemacht worden ist. Nach Einbringen der Arzneistoffschüttung 9 in den Preßraum 8 wird die Presse mit Öldruck beaufschlagt (vgl. Pfeilrichtung in Fig. 1a), so daß sich der in Fig. 1a gezeigte Zustand einstellt.

Wie in Fig. 1a gut zu erkennen ist, führen die Platten 2 keinen Stempelweg aus und üben somit auch keinen aktiven Druck auf die Arzneistoffschüttung aus. Vielmehr nehmen diese den Druck auf, der durch das Hydraulikmedium 5 über die Druckmembran 7 und die Preßmatritze 8 in radialer Richtung aufgebracht wird. Durch das konzentrische Aufbringen des Hydraulikdruckes in radialer Richtung in Richtung auf den Kern 3 zu wird die Arzneistoffschüttung verdichtet und ein verfestigter Arzneistoffvorrat erzeugt. Bei diesem Preßvorgang rutscht die Preßmatritze auf den Stirnflächen der oberen und unteren Platte 2 etwas in radialer Richtung nach innen, so daß die Druckübertragung auf den Arzneistoffvorrat 9a ausschließlich in radialer Richtung erfolgt und jede axiale Scherbewegung innerhalb des Arzneistoffvorrates vermieden ist.

Ein derart erzeugter Arzneistoffvorrat besitzt infolge der eingangs erwähnten inneren Reibung einen Dichtegradienten, der streng radial ausgerichtet ist, d.h. die Zonen gleicher Dichte verlaufen parallel und konzentrisch um die Mittelachse des Arzneistoffvorrates 9a.

Nach dem Pressen wird der Öldruck abgebaut, die Preßmatritze 8 und die Druckmembran 7 kehren in ihre Ausgangsposition (Fig. 1) zurück und der erzeugte Arzneistoffvorrat in Form einer Ringtablette 9a wird nach dem Öffnen der Preßform entnommen. Technische Einzelheiten der Preßkammer, wie beispielsweise die Abdichtung der Ölfüllung und dergleichen, sind aus Gründen der Übersichtlichkeit nicht dargestellt.

Der oben beschriebene ringförmige Arzneistoffvorrat kann beispielsweise einen Außendurchmesser von 16 mm, einen Innendurchmesser von 10 mm und eine Höhe von 6 bis 60 mm aufweisen. Der Arzneistoff kann beispielsweise eine Mischung von 7,5% Salbutamol in granulierter Laktose sein, der mit einem Preßdruck von 170 MPa gepreßt wird. Hierdurch ergibt sich in axialer Richtung bei einer integralen Dichte von 1,317 g/cm⁻³ ein Dichtegradient von nicht mehr als 0,05 % und in radialer Richtung ein Gradient von nicht mehr als 0,3%, wobei das Dichteminimum etwa in der Mitte der Wandstärke der Ringtablette liegt. Mit diesen Eigenschaften ist ein derartig hergestellter Arzneistoffvorrat optimal für einen Aerosolgenerator angepaßt, wie er in der bereits genannten WO 93/24165 beschrieben ist, da in diesem Gerät das Abtragen der Partikel von dem Arzneistoffvorrat durch eine in axialer Richtung einwirkende Stirnfräse erfolgt und hierbei stets über den gesamten Verbrauchsbereich eine insgesamt sehr geringe, aber gleichartige, konzentrische Dichteverteilung vorgefunden wird, die somit keinen Einfluß auf die Aerosolmenge und Qualität besitzt. Wesentlich ist ferner, daß der Preßvorgang ohne jedes Preßhilfsmittel durchgeführt werden kann. Der z.B. ringförmige Arzneistoffvorrat gemäß der Erfindung kann in einfacher Weise in dem Tablettenhalter eines Aerosolgenerators mittels Cyanoacrylatkleber befestigt werden.

Es sei nochmals festgestellt, daß die obengenannten Daten und Stoffe des Arzneistoffvorrates lediglich rein beispielhaft genannt sind und sich die vorliegende Erfindung auf eine Vielzahl von Arzneistoffen bzw. Arzneistoffmischungen anwenden läßt. Hierbei wird unter Arzneistoff jede pharmazeutische Zusammensetzung verstanden.

## Patentansprüche

1. Verfestigter Arzneistoffvorrat für die Erzeugung von inhalierbaren Arzneistoffpartikeln mittels einer Abtragemittel aufweisenden Dosiereinrichtung,
dadurch gekennzeichnet, daß das Gefüge des Arzneistoffvorrats durch Aufbringen eines radialen Preßdrucks von außen auf einen im Zentrum des Arzneistoffvorrats positionierten Kern hergestellt ist.

2. Verfestigter Arzneistoffvorrat nach Anspruch 1,
dadurch gekennzeichnet, daß dieser einen kreisförmigen Querschnitt aufweist.

3. Arzneistoffvorrat nach zumindest einem der vorstehenden Ansprüche,
dadurch gekennzeichnet, daß dieser aus einer granulierten Trägerstoff-Wirkstoffmischung, vorzugsweise mit einem Preßdruck zwischen 50 und 500 MPa, hergestellt ist.

4. Arzneistoffvorrat nach zumindest einem der vorstehenden Ansprüche,
dadurch gekennzeichnet, daß dieser einen Dichtegradienten aufweist, der senkrecht zur Richtung der Abtragung nicht mehr als 0,3% beträgt und der vorzugsweise in Richtung der Abtragung nicht mehr als 0,05% beträgt.

5. Verfahren zur Herstellung eines verfestigten Arzneistoffvorrats für die Erzeugung von inhalierbaren Arzneistoffpartikeln mittels einer Abtragemittel aufweisenden Dosiereinrichtung, wobei auf den Arzneistoffvorrat ein Preßdruck aufgebracht wird,
dadurch gekennzeichnet, daß der Preßdruck radial von aussen auf einen im Zentrum des Arzneistoffvorrats positionierten Kern gerichtet ist.

6. Verfahren nach Anspruch 5,
dadurch gekennzeichnet, daß der Arzneistoffvorrat vorgeformt dem Preßdruck ausgesetzt wird.

7. Verfahren nach zumindest einem der Ansprüche 5 oder 6,
dadurch gekennzeichnet, daß der Arzneistoffvorrat als Ringkörper vorgeformt ist.

8. Verfahren nach zumindest einem der Ansprüche 5 bis 7,
dadurch gekennzeichnet, daß der Kern nach dem Pressen entfernt wird.

## Claims

1. Solidified supply of pharmaceutical agent for production of inhalable particles of pharmaceutical agent by means of a dosing device with a substance removal feature,
characterized by structuring of the supply of pharmaceutical agent by applying radial pressing pressure from the outside to a core positioned in the centre of the supply of pharmaceutical agent.

2. Solidified supply of pharmaceutical agent as per Claim 1,
characterized by a ring-shaped cross-section.

3. Supply of pharmaceutical agent acc. to at least one of the previous Claims,
characterized by the supply consisting of a granulated mixture of carrier substance and active ingredient, preferably formed under pressure between 50 and 500 MPa.

4. Supply of pharmaceutical agent acc. to at least one of the previous Claims,
characterized by a gradient of density of the supply amounting to no more than 0.3% vertical to the direction of substance removal and preferably no more than 0.05% in the direction of substance removal.

5. Method of production of a solidified supply of pharmaceutical agent for production of inhalable particles of pharmaceutical agent by means of a dosing device with a substance removal feature, whereby pressure is applied to the supply of pharmaceutical agent,
characterized by application of the pressure radially from the outside, directed at a core positioned in the centre of the supply of pharmaceutical agent.

6. Method as per Claim 5,
characterized by exposure of the supply of pharmaceutical agent to the pressure in pre-formed state.

7. Method acc. to at least one of Claims 5 or 6,
characterized by pre-forming of the supply of pharmaceutical agent in a ring shape.

8. Method acc. to at least one of Claims 5 to 7,
characterized by removal of the core after pressure application.

## Revendications

1. Réserve à substance médicamenteuse consolidée destinée à la production de particules médicamenteuses à inhaler à l'aide d'un dispositif de dosage présentant un système d'abrasion, caractérisé par le fait que l'assemblage de la réserve à substance médicamenteuse est réalisé par l'application radiale d'air comprimé de l'extérieur sur un noyau positionné au centre de la réserve à substance médicamenteuse.

2. Réserve à substance médicamenteuse consolidée selon la revendication 1, caractérisée par le fait que cette réserve présente une section circulaire.

3. Réserve à substance médicamenteuse selon au moins une des revendications précédentes, caractérisée par le fait que cette réserve est fabriquée à partir d'un mélange granuleux de principes actifs et d'une substance porteuse, de préférence avec de l'air comprimé compris entre 50 et 500 Mpa.

4. Réserve à substance médicamenteuse selon au moins une des revendications précédentes, caractérisée par le fait que cette réserve présente un gradient de densité qui, perpendiculairement au sens de l'abrasion, ne s'élève pas à plus de 0,3 % et qui, de préférence dans le sens de l'abrasion, ne dépasse pas plus de 0,05 %.

5. Procédé de fabrication d'une réserve à substance médicamenteuse consolidée destinée à la production de particules médicamenteuses pouvant être inhalées à l'aide d'un dispositif de dosage présentant un système d'abrasion, de l'air comprimé étant appliqué sur la réserve, caractérisé par le fait que l'air comprimé est dirigé radialement de l'extérieur sur un noyau positionné au centre de la réserve à substance médicamenteuse.

6. Procédé selon la revendication 5, caractérisé par le fait que la réserve à substance médicamenteuse préformée est exposée à l'air comprimé.

7. Procédé selon au moins une des revendications 5 ou 6, caractérisé par le fait que la réserve à substance médicamenteuse est préformée en forme d'anneau.

8. Procédé selon au moins une des revendications 5 à 7, caractérisé par le fait que le noyau est retiré après le pressage.
